(19) Europäisches Patentamt
European Patent Office
Office européen des brevets

(11) Numéro de publication: **0 236 164**
**B1**

(12) # FASCICULE DE BREVET EUROPEEN

(45) Date de publication du fascicule du brevet: **12.09.90**

(21) Numéro de dépôt: **87400150.6**

(22) Date de dépôt: **22.01.87**

(51) Int. Cl.[5]: **C 07 C 311/19,** C 07 K 5/06, A 61 K 37/02

(54) **Dérivés des N alpha-arylsulfonylaminoacyl p-amidino-phénylalaninamides, leur procédé de préparation, leur application comme médicaments et leurs intermédiaires de synthèse.**

(30) Priorité: **24.01.86 FR 8601399**
**24.01.86 FR 8601400**

(43) Date de publication de la demande:
**09.09.87 Bulletin 87/37**

(45) Mention de la délivrance du brevet:
**12.09.90 Bulletin 90/37**

(84) Etats contractants désignés:
**AT BE CH DE ES FR GB GR IT LI LU NL SE**

(56) Documents cités:
**CHEMICAL ABSTRACTS, vol. 98, 1983, page 645, résumé no. 107770b, Columbus, Ohio, US; & DD-A-155 954 (G. WAGNER et al.) 21-07-1982**

**CHEMICAL ABSTRACTS, vol. 99, 1983, page 279, résumé no. 18600z, Columbus, Ohio, US; J. STUERZEBECHER et al.: "Cyclic amides of Nalpha-arylsulfonylaminoacylated 4-amidinophenylalanine - light binding inhibitors of thrombin", & THROMB. RES. 1983, 29(6), 635-42**

(73) Titulaire: **SANOFI**
**40, Avenue George V**
**F-75008 Paris (FR)**

(72) Inventeur: **Bernat, André**
**14 Chemin Maurens**
**F-31270 Cugnaux (FR)**
Inventeur: **Delebassée, Denis**
**276 Chemin des Crêtes**
**Goyrans F-31120 Portet/Garonne (FR)**
Inventeur: **Frehel, Daniel**
**Résidence l'Autan Appt. 206 100 Allée de Barcelone**
**F-31000 Toulouse (FR)**
Inventeur: **Maffrand, Jean-Pierre**
**5 Rue du Corps Franc Pommiès**
**F-31120 Portet/Garonne (FR)**
Inventeur: **Vallée, Eric**
**253 Chemin du Ramelet Moundi**
**F-31170 Tournefeuille (FR)**

(74) Mandataire: **Bressand, Georges et al**
**c/o CABINET LAVOIX 2 Place d'Estienne d'Orves**
**F-75441 Paris Cédex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 236 164 B1

Courier Press, Leamington Spa, England.

(56) Documents cités:
CHEMICAL ABSTRACTS, vol. 102, 1985, page 229, résumé no. 91802b, Columbus, Ohio, US; U. GRIESSBACH et al.: "Assay of hirudin in plasma using a chromogenic thrombin substrate", & THROMB. RES. 1985, 37(2), 347-50

CHEMICAL ABSTRACTS, vol. 104, 1986, page 43, résumé no. 475z, Columbus, Ohio, US; J. HAUPTMANN et al.: "Anticoagulant action of synthetic tight binding inhibitors of thrombin in vitro and in vivo", & THROMB. RES. 1985, 39(6), 771-5

CHEMICAL ABSTRACTS, vol. 104, 1986, page 31, résumé no. 45508d, Columbus, Ohio, US; B. KASIER et al.: "Pharmacological characterization of a new highly effective synthetic thrombin inhibitor", & BIOMED.

BIOCHIM. ACTA 1985, 44(7-8), 1201-10

DIE PHARMAZIE, vol. 40, no. 5, May 1985, pages 305-306, VEB Verlag Volk und Gesundheit, Berlin, DE; B. VOIGT et al.: "Synthese von Nalpha-Benzyloxycarbonyl-4-amidinophenylalaninamiden als Thrombininhibitoren"

**Description**

La présente invention est relative à de nouveaux dérivés Nα-arylsulfonylaminoacyl p-amidino-phénylalaninamides, à leur procédé de préparation et à leur application en tant qu'agents inhibiteurs sélectifs de la thrombine et antithrombotiques.

Les composés de l'invention répondent à la formule générale (I):

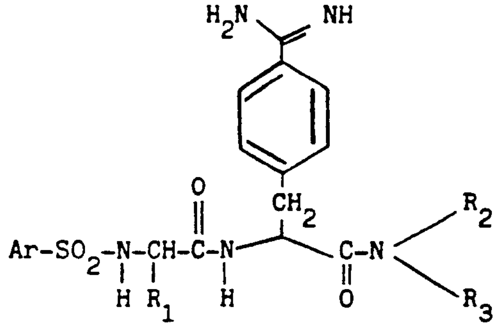

(I)

dans laquelle:

$R_1$ représente un groupe alcoyle en $C_1$—$C_6$ hydroxyalcoyle en $C_1$—$C_6$ benzyle, un groupe phényle ou un groupe hydroxy-4 phényle;

$R_2$ et $R_3$, identiques ou différents, représentent chacun un radical alcoyle, hydroxyalcoyle, alcényle, ayant jusqu'à 6 atomes de carbone, ou forment ensemble avec l'azote auquel ils sont attachés, un hétérocycle saturé choisi parmi morpholino, thiomorpholino, pyrrolidino non substitué ou substitué par un groupe alcoxy en ($C_1$—$C_6$) carbonyle ou carboxyle, pipérazino, (alcoyle en $C_1$—$C_6$)-4 pipérazino, (hydroxyalcoyle en $C_1$—$C_6$)-4 pipérazino ou pipéridino non substitué ou substitué par un groupe alcoyle en $C_1$—$C_6$, benzyle, hydroxy, hydroxyalcoyle en $C_1$—$C_6$ amino, aminoalcoyle en $C_1$—$C_6$ alcoxy en $C_1$—$C_6$ carbonyle ou carboxyle;

Ar représente un gorupe phényle, alpha-naphtyle ou béta-naphtyle ou bien un groupe hétéroaryle choisi parmi pyridyle, quinoléinyle, isoquinoléinyle. Les composés de formule I ci-dessus préférés sont ceux dans lesquels $R_1$ représente un radical alcoyle ou hydroxyalcoyle, ceux dans lesquels le groupe

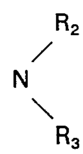

représente un radical piperidino ou methyl-4 pipéridino, et ceux dans lesquels $A_r$ représente un radical naphtyle. Le carbone porteur du groupe $R_1$ peut avoir la configuration R ou S ou RS. Tous les composés présentant les dites configurations sont compris dans la présente invention.

Les composés de formule (I) ci-dessus, comportant un ou plusieurs centres asymétriques peuvent exister sous forme de plusieurs isomères (diastéréoisomères, énantiomères) et la cristallisation peut entraîner un enrichissement de certains des diastéréoisomères. L'invention concerne aussi bien chaque stéréoisomère que leurs mélanges. L'invention comprend aussi des sels d'addition des composés de formule (I) avec les acides minéraux ou organiques pharmaceutiquement acceptables.

Les termes "alcoyle inférieur", "alcényle inférieur", et "alcynyle inférieur" tels qu'utilisés ici, désignent les radicaux d'hydrocarbures aliphatiques ramifiés ou linéaires, contenant jusqu'a six atomes de carbone, tels que méthyle, éthyle, isopropyle, isobutyle, tertiobutyle, n-hexyle, allyle, propargyle, crotyle, méthyl-2 crotyle, méthyl-2 allyle, butyryle-2. Des inhibiteurs de thrombine synthétiques, présentant un groupe amidinophénylalanine ont été décrits dans la littérature.

G. WAGNER et ses collaborateurs (DD Patent 142804 (16.07.80) ont décrit les composés de formule générale (A):

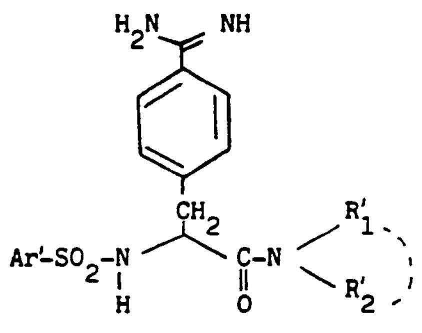

(A)

L'insertion d'un résidu aminoacide glycine entre le groupe sulfonyle et l'azote N- α de la p-amidinophénylalanine a conduit à des composés de formule générale (B) dont l'activité in vitro est potentialisée par rapport à ceux de formule générale (A) (G. WAGNER et Coll. DD Patent 155954 (03.02.81)).:

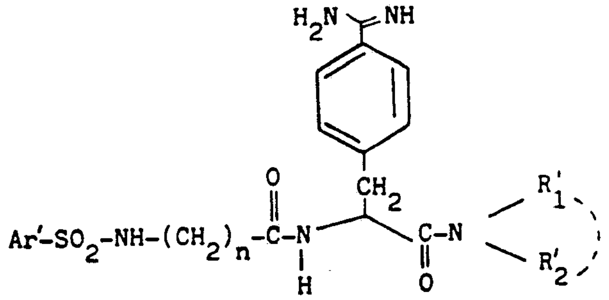

(B)

et parmi ceux-ci, le composé de formule (B) où n = 1, Ar' = β-naphtyle, $NR_1'R'_2$ = pipéridino, ci-après désigné composé (C) présente la meilleure activité inhibitrice de la thrombine in vitro (J. STURZEBECHER et al. Thrombosis Research 1983, *29*, 635) et ex vivo (J. HAUPTMANN et al. Thrombosis Research 1985, *39*, 771).

Les produits de formule (A) et (B) ci-dessus sont préparés selon les procédés décrits dans les brevets DDR 142804 et DDR 155954, les amides sont préparés à partir des acides libres correspondants par activation et réaction avec l'amine correspondante. Ces procédés impliquent des conditions de réactions qui induisent des racémisations au niveau du centre asymétrique. En outre, ils ne permettent pas d'obtenir des composés portant le substituant $R_1$.

La demanderesse a trouvé que les composés de formule (I) ci-dessus, peuvent être obtenus par un procédé qui permet, par l'utilisation de procédés de couplages et de groupements protecteurs, judicieusement choisis, de respecter les centres d'asymétrie dans leur configuration originelle et qui n'induit pas de racémisation.

Ce résultat est obtenu, contrairement aux procédés décrits par G. WAGNER et ses collaborateurs, en construisant d'abord la partie

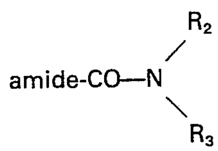

à partir de la fonction acide du synthon p-cyanophénylalanine, avant la partie arylsulfonylaminoacyle.

L'invention a également pour objet un procédé de préparation des composés de formule (I) caractérisé en ce que l'on fait réagir sur la cyano-4 phénylalaninamide de formule (II):

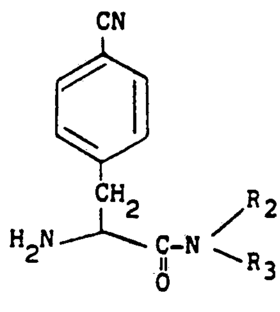

(II)

dans laquelle $R_2$ et $R_3$ ont les mêmes significations que dans la formule (I) un acide de formule

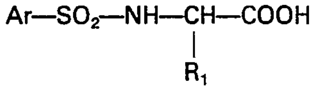

(III)

sous sa forme activée (IV):

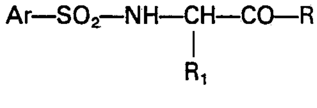

(IV)

dans laquelle Ar et $R_1$ ont les mêmes significations que dans la formule (I) et R représente un bon groupement nucléofuge, tel que chloro, alcoxycarbonyloxy ou hétéroaryle pour obtenir le composé de formule (V)

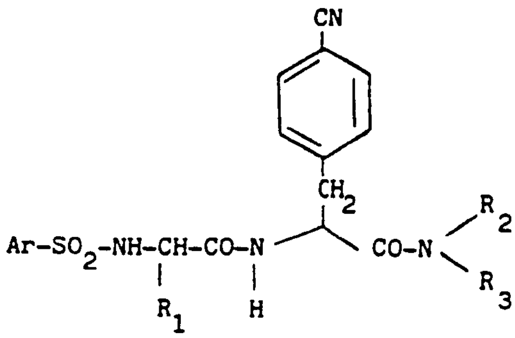

(V)

dans laquelle Ar, $R_1$, $R_2$ et $R_3$ ont les mêmes significations que dans la formule (I), qu'on traite avec un excès d'une solution saturée de gaz chlorhydrique dans un alcool de formule X—OH dans laquelle X représente un radical alcoyle inférieur, pour obtenir le composé de formule (VI) sous forme de chlorhydrate:

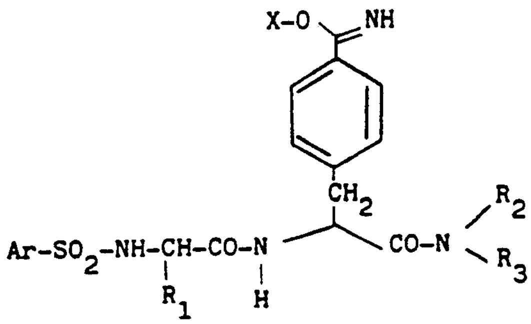

(VI)

dans lequel Ar, $R_1$, $R_2$, $R_3$ et X ont les mêmes significations précitées. L'imido-ester de formule (VI) est alors traité par un excès d'une solution de gaz ammoniac dans un alcool inférieur à la température d'ébullition du mélange réactionnel pour obtenir le composé de formule (I) recherché.

Ce composé est isolé sous forme de sel, la base libre pouvant être obtenue par les procédés classiques et éventuellement transformée en un autre sel pharmaceutiquement acceptable tel que par exemple, outre le chlorhydrate, le bromhydrate, le sulfate, le méthanesulfonate, l'acétate, le naphtalènesulfonate-2, le maléate, le fumarate, le citrate, le gluconate, le dobésilate ou le sultosilate.

La préparation du nouveau composé de formule (II) s'effectue à partir de la cyano-4 phénylalanine de formule suivante et sera décrite plus loin.

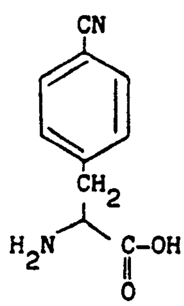

L'acide de formule

$$Ar-SO_2-NH-\underset{\displaystyle R_1}{\underset{|}{CH}}-COOH \qquad (III)$$

a été préparé selo le schéma réactionnel ci-après:

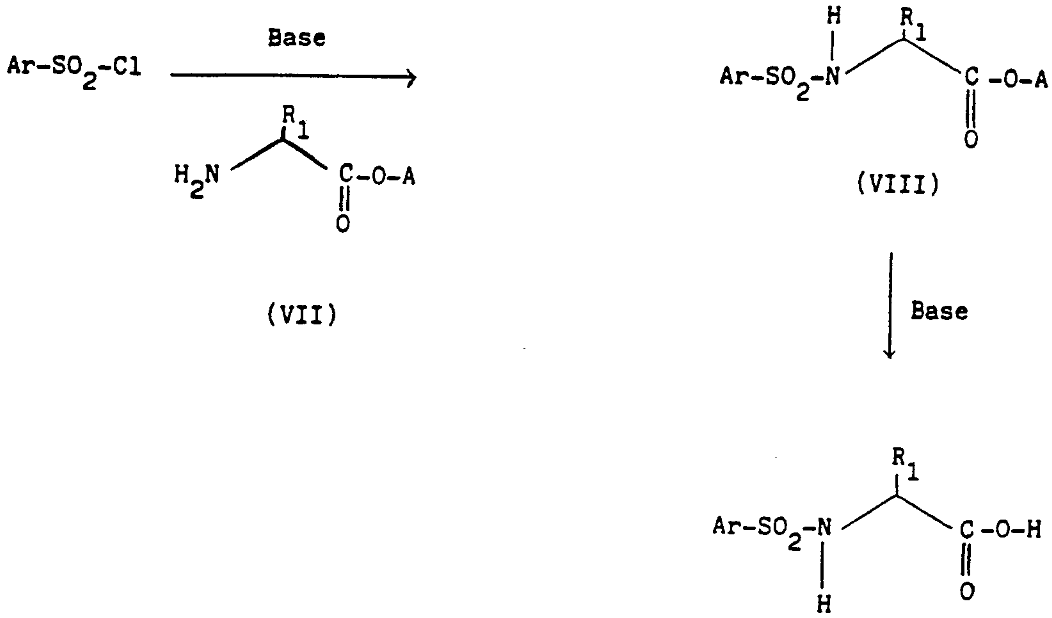


(VII)

(VIII)

(III)

L'introduction d'un centre asymétrique dans l'aminoester (VII, A = alcoyle inférieur) dont la configuration "R" ou "S" initiale doit être conservée jusqu'à l'acide (III), nécessite l'emploi de méthodes non racémisantes:

la sulfonylation de l'aminoester (VII) s'opère en milieu biphasique, de préférence le mélange eau-dichlorométhane, eau-chloroforme, eau-tétrachlorure de carbone, en présence d'une base, de préférence un carbonate alcalin tel que le carbonate de potassium, le carbonate de sodium, à des températures comprises entre 10°C et 25°C.

la saponification de l'ester (VIII) s'opère en milieu hydroalcoolique tel que eau-méthanol ou eau-éthanol, en présence d'un équivalent d'hydroxyde alcalin, de préférence l'hydroxyde de sodium, à des températures comprises entre 10°C et 25°C. La neutralisation du milieu réactionnel par addition d'un équivalent d'une solution acqueuse 1N d'acide minéral, de préférence l'acide chlorhydrique, conduit à l'acide (III). Cette saponification peut également être menée à bien dans un milieu hydro-organique, tel que eau-dioxanne dans les mêmes conditions. Dans le cas où il n'est pas nécessaire de conserver la configuration du carbone asymétrique, on pourra utiliser une méthode classique. Pour le transformation de l'acide de formule (III) en une forme activée de formule (IV), deux cas sont à envisager:

a) *Cas où le carbone porteur du groupe $R_1$ possède la configuration "RS" (racémique) :* (synthèse non stéréospécifique)

On peut utiliser indifféremment l'activation de la fonction acide par exemple par:

transformation de la fonction acide en halogénure d'acyle (IV : R = Cl):

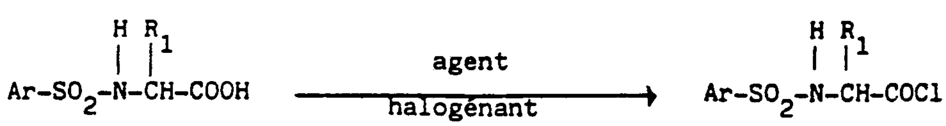


(III) (IV : R = Cl)

selon le procédé décrit dans le brevet DDR 155954.

transformation de la fonction acide en anhydride carbonique mixte

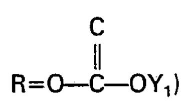

selon le schéma réactionnel:

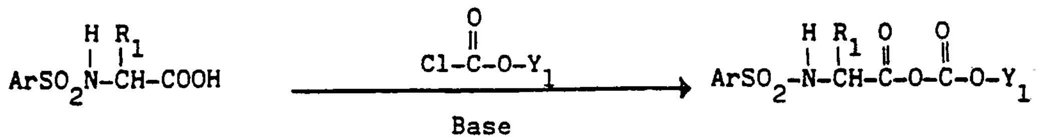


(III) (IV : R = $O-C-O-Y_1$)

La réaction utilise un chloroformate d'alcoyle

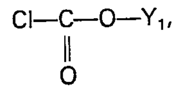

où $Y_1$ est un radical alcoyle inférieur ramifié ou non, en présence d'une amine tertiaire comme base. Le chloroformate d'alcoyle préférentiellement utilisé est le chloroformate d'éthyle ($Y_1 = C_2H_5$) ou d'isobutyle ($Y_1 = CH_2—CH(CH_3)_2$).

L'amine tertiaire préférentielle est la triéthylamine. Cette condensation s'opère de préférence à des températures comprises entre −5°C et +10°C, dans un solvant inerte tel que le dichlorométhane, le chloroforme ou le tétrachlorure de carbone.

Les composés obtenus de formule (IV) sont mis à réagir avec les composés de formule (II), dans un solvant inerte, en présence d'un accepteur d'acides tel qu'une amine tertiaire.

b) *Cas où le carbone porteur du groupe $R_1$ possède la configuration "R" ou "S" (énantiomères R ou S)* (Synthèse stéréospécifique)

Il est nécessaire dans ce cas, d'utiliser des procédés n'induisent pas de racémisation.

La transformation de la fonction acide des composés de formule (III) en esters activés, conduit à des composés de formule générale (IV : R = O—Z) selon le schéma réactionnel:

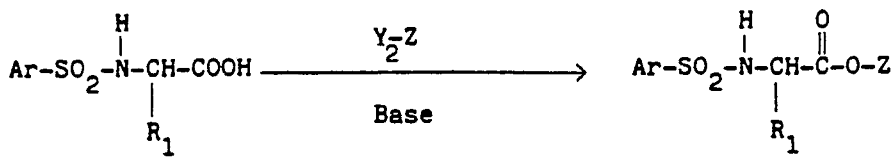


(III) (IV : R = O-Z)

Les réactifs de couplage $Y_2$—Z, n'induisant pas de racémisation, utilisés de préférence, mais non limitatifs, sont les suivants:

Hydroxy-1 benzotriazole (HOBT) ($Y_2$ = OH; Z = 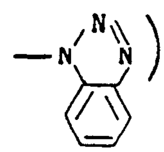)

en présence de N,N-dicyclohexylcarbodiimide (DCC) selon le mode opératoire décrit par E. C. JORGENSEN et al. (J. Am. Chem. Soc. 1971, *93*, 6318).

Hexafluorophosphate de benzotriazolyl-1 oxytris (diméthylamino) phosphonium (BOP)

($Y_2 = [(CH_3)_2N]_3 \overset{\oplus}{P}{=}O\ PF_6^{\ominus}$ ; Z = 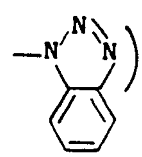)

selon le mode opératoire décrit par B. CASTRO et Coll. Synthesis 1976, 751.

Chlorure de N,N-bis (oxo-2 oxazolidinyl-3) phosphorodiamidique selon le mode opératoire décrit par D. H. RICH et al. J. Am. Chem. Soc. 1985, *107*, 4342.

La réaction d'activation et de couplage s'opère en présence d'amines tertiaires, de préférence la triéthylamine, dans un solvant inerte tel que le dichlorométhane, le diméthylformamide ou l'acétonitrile, à des températures comprises entre 15°C et 40°C.

La formation de l'imidoester (VI) s'effectue en milieu alcoolique tel que le méthanol ou l'éthanol, à une température comprise entre −10°C et +10°C, de préférence à 0°C pendant une durée de 16 h à 24 h.

L'amidine de formule (I) est obtenue en traitant le composé VI) précédemment obtenu sans autre purification, dans une solution alcoolique de gaz ammoniac à une normalité de 3N à 15 N, à la température ambiante et on chauffe ensuite le mélange au reflux pendant 1 à 3 h.

Les composés de formule (II) ci-dessus, comportant un centre asymétriques, peuvent exister sous forme de deux isomères (énantiomères).

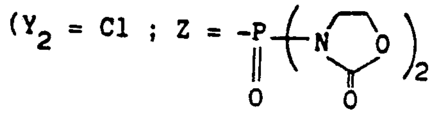

Les composés de formule (II) sout préparés par réaction de l'acide aminé de formule (IX) sous sa forme activée (X):

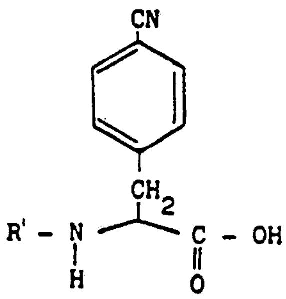
(IX)

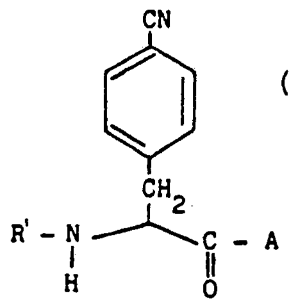
(X)

dans lesquelles R' représente un groupement N-protecteur et A représente le reste d'un réactif de couplage, avec l'amine de formule (XI):

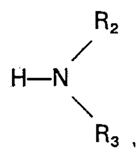

dans laquelle $R_2$ et $R_3$ sont tels que décrits dans la formule (II) pour former le composé de formule (XII):

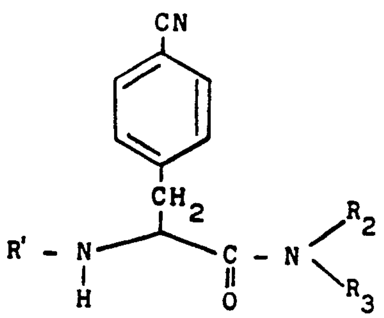
(XII)

dans laquelle R', $R_2$ et $R_3$ ont les mêmes significations que dans les formules (II) et (IX) et qui, par clivage de groupement protecteur R', conduit aux composés de formule (II).

La formation du composé de formule (IX) est obtenue par fixation du groupement N-protecteur R' sur la p-cyanophénylalanine de formule:

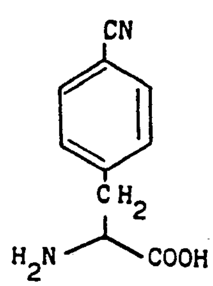

Le groupe N-protecteur, représenté par R', est un des groupements stables en milieu alcalin, utilisés pour la protection des groupes amino des acides aminés dans la chimie des peptides, par exemple le groupe

terbutyloxy carbonyle, de préférence désigné ci-après Boc; le groupe (diméthoxy-3,5 phényl)-2 propyl-2 oxycarbonyle désigné comme Ddz; le groupe (biphényl-4yl)-2 propyl-2 oxycarbonyle désigné comme Bpoc; le groupe (nitro-2 phényl)sulfényle désigné comme Nps.

Pour obtenir l'acide activé de formule (X) dans lequel A représente le reste du réactif de couplage, deux cas sont à envisager.

a) *Procédé de préparation avec conservation de la configuration "R" ou "S"* (synthèse stéréospecifique)

Pour ne pas indurie de racémisation au niveau du centre asymétrique du composé de formule (XII) et conserver la configuration initiale du centre asymétrique de l'acide de formule (IX), il est nécessaire d'employer une activation de l'acide (IX), utilisant la transformation de la fonction acide en ester activé (X) suivant le schéma réactionnel:

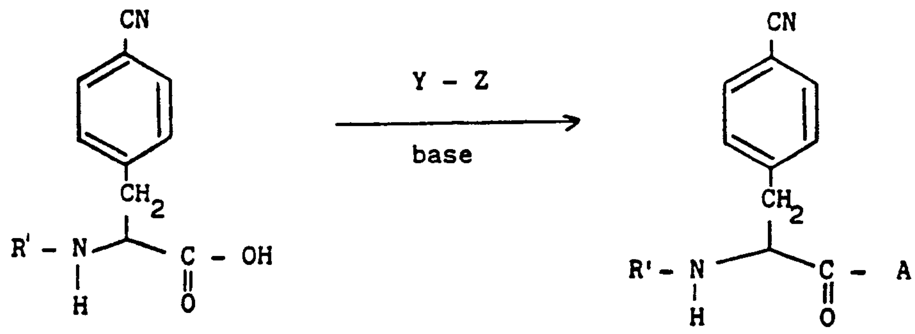


(IX) ( X)

Les réactifs de couplage Y—Z, n'induisant pas de racémisation, utilisés de préférence, mais non limitatifs, sont les suivants:

Hydroxy-1 benzotriazole (HOBT)

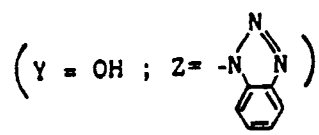


en présence de N,N-dicyclohexylcarbodiimide (DCC) selon le mode opératoire décrit par E. C. JORGENSEN et al. (J. Am. Chem. Soc. 1971, *93,* 6318).

Hexafluorophosphate de benzotriazolyl-1 oxytris (diméthylamino) phosphonium (BOP)

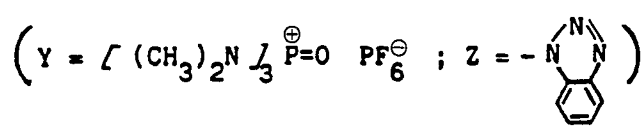

selon le mode opératoire décrit par B. CASTRO et al. (Synthesis 1976, 751).

Chlorure de N,N-bis (oxo-2 oxazolidinyl-3) phosphorodiamidique

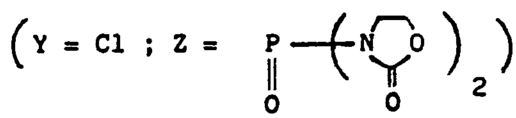


selon le mode opératoire décrit par D. H. RICH et al. (J. Am. Soc. 1985, *107,* 4342).

La réaction d'activation et de couplage s'opère en présence d'amines tertiaires, de préférence la triéthylamine, dans un solvant inerte tel que le dichlorométhane, le diméthylformamide ou l'acétonitrile, à des températures comprises entre 15°C et 40°C.

b) *Procédé de préparation sans conservation de la configuration* (synthèse non stéréospécifique)

On peut effectuer l'activation de la fonction acide du composé (IX) par transformation de la fonction acide en anhydride carbonique mixte

$(X : A = -O-\underset{\underset{O}{\|}}{C}-O-B)$ selon le schéma réactionnel:

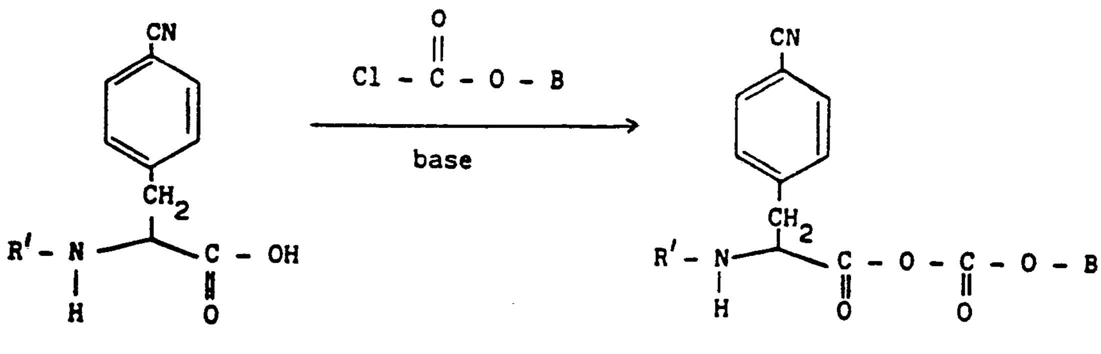


(IX) (X')

La réaction utilise un chloroformate d'alcoyle

$$Cl-\underset{\underset{\displaystyle O}{\|}}{C}O-B,$$

où B est un alcoyle inférieur ramifié ou non, en présence d'une amine tertiaire comme base. Le chloroformate d'alcoyle préférentiellement utilisé est le chloroformate d'éthyle ($B = C_2H_5$) ou d'isobutyle ($B = CH_2—CH(CH_3)_2$).

L'amine tertiaire préférentielle est la triéthylamine. Cette condensation s'opère de préférence à des températures comprises entre =5°C et +10°C, dans un solvant inerte tel que le dichlorométhane, le chloroforme ou le tétrachlorure de carbone.

La p-cyanophénylalanine de départ a été préparée selon une des méthodes utilisées dans la littérature (G. WAGNER et al. Pharmazie 1981, *36* (9), 597).

Le composé de formule (X) est mis à réagir avec l'amine de formule (XI) dans un solvant inerte et en présence d'une amine tertiaire.

Le clivage du groupe N-protecteur R' du composé de formule (XII) conduit aux p-cyanophénylalaninamides Nα-substituées de formule (II). Ce clivage s'opère en milieu acide, de préférence un mélange acide bromhydrique-acide acétique ou bien dans l'acide trifluoroacétique (R' = Boc, Ddz, Bpoc, Nps), dans l'acide acétique (R' = Boc, Nps), dans une solution saturée de gaz chlorhydrique dans l'acétate d'éthyle (R'=Boc), à des températures comprises entre 0° et 20°C.

## Exemple 1

*Nα-(terbutyloxycarbonyl) p-cyanophénylalanine*

$$(IX:\ R'=—\underset{\underset{\displaystyle O}{\|}}{C}—OC(CH_3)_3)$$

On dissout 10 g (0,044 mole) de chlorhydrate de p-cyanophénylalanine dans 220 ml de dioxanne et 88,2 ml (0,088 mole) d'hydroxyde de sodium aqueux 1N. A température ambiante et sous atmosphère inerte, on ajoute par portions, au milieu réactionnel, 1,77 g (0,044 mole) d'oxyde de magnésium, puis 10,6 g (0,0484 mole) de dicarbonate de diterbutyle.

On agite à température ambiante, pendant 20 h. On filtre les cristaux qui sont lavés avec de l'eau. On évapore le filtrat et dissout le résidu dans l'eau. La phase aqueuse obtenue est amenée à pH = 3 par addition d'une solution saturée d'hydrogénosulfate de potassium. On extrait la phase aqueuse avec 2 × 400 ml d'acétate d'éthyle, sèche les extraits organiques sur sulfate de sodium anhydre et évapore à sec. Les cristaux obtenus sont recristallisés dans l'acétate d'éthyle ou l'éther diisopropylique.
Cristaux blancs, rendement: 88%, F = 147°C.

## Exemple 2

*[Nα-(terbutyloxycarbonyl) p-cyanophénylalanyl]-1 pipéridine*

$$(XII:\ R=—\underset{\underset{\displaystyle O}{\|}}{C}—OC(CH_3)_3;$$

$NR_2R_3$ = pipéridino). Activation de la fonction acide du composé

(IX: R=—C—$OC(CH_3)_3$)
||
O

(exemple 1) en anhydride carbonique mixte

(X: A=—C—O—C—$OC_2H_5$)
|| ||
O O

On ajoute à 0°C, sous atmosphère inerte, 4,2 g (0,0416 mole) de triéthylamine à une suspension de 11 g (0,0378 mole) de Nα-(terbutyloxycarbonyl) p-cyanophénylalanine (exemple 1) dans 125 ml de dichlorométhane. Au mélange devenu homogène, on ajoute, goutte à goutte, une solution de 4,3 g (0,0395 mole) de chloroformate d'éthyle dans 10 ml dichlorométhane. Après la fin de l'addition, on abandonne 45 minutes le mélange réactionnel, à 0°C, puis ajoute, goutte à goutte, 3,4 g (0,0397 mole) de pipéridine dissoute dans 10 ml de dichlorométhane. On laisse revenir le milieu réactionnel à température ambiante et abandonne pendant 15 h à cette température. On extrait le milieu réactionnel avec une solution aqueuse saturée de bicarbonate de sodium. La phase organique, après décantation, est séchée sur sulfate de sodium anhydre et évaporée à sec. Le résidu huileux donne des cristaux blancs, après trituration avec de l'éther diisopropylique. Ces cristaux sont recristallisés dans l'éther diisopropylique.
Cristaux blancs, rendement: 81%, F = 132°C.

Exemple 3

*méthyl-4 [Nα-(terbutyloxycarbonyl) p-cyanophénylalanyl]-1 pipéridine*

(XII: R=—C—$OC(CH_3)_3$;
||
O

$NR_2R_3$ = méthyl-4 pipéridino). Activation par transformation de la fonction acide en une fontion ester activé, par utilisation du réactif de couplage non racémisant DCC/HOBT.

On dissout 2,78 g (0,01 mole) de Nα-(terbutyloxycarbonyl) p-cyanophénylalanine (exemple 1) dans 50 ml de dichlorométhane. Sous atmosphère inerte, à température ambiante, on ajoute successivement 1 g (0,01 mole) de méthyl-4 pipéridine, 1,35 g (0,01 mole) d'hydroxy-1 benzotriazole (HOBT), 1,1 g (0,01 mole) de triéthylamine. Au milieu réactionnel, on ajoute, à température ambiante, 2,06 g (0,01 mole) de N,N-dicyclohexylcarbodiimide (DDC) dissoute dans 80 ml de dichlorométhane et abandonne le milieu réactionnel pendant 15 h à température ambiante. On filtre le précipité de dicyclohexylurée que l'on élimine. Le filtrat organique est lavé avec une solution aqueuse saturée de bicarbonate de sodium, est séché sur sulfate de sodium anhydre. L'évaporation laisse un résidu qui est trituré avec de l'éther diisopropylique. Les cristaux blancs obtenus sont recristallisés dans l'acétate d'éthyle.
Cristaux blancs, rendement: 84%, F=142°C (acétate d'éthyle).

Exemple 4

*(p-cyanophénylalanyl)-1 pipéridine* (II: $NR_2R_3$=pipéridino)

A 22 ml d'une solution saturée de gaz chlorhydrique dans l'acétate d'éthyle, refroidie à 0°C, on ajoute 4,1 g (0,0115 mole) de [Nα-(terbutyloxycarbonyl) p-cyanophénylalanyl]-1 pipéridine (exemple 1) dissoute dans 22 ml d'acétate d'éthyle. Il y a dissolution progressive. On laisse revenir à température ambiante et abandonne 15 h. à cette température.

Après 2 h, les cristaux précipitent, ils sont filtrés, lavés à l'éther et recristallisés dans l'isopropanol.

Cristaux blancs, rendement: 88%, F=145°C, chlorhydrate.

Exemple 5

*(p-cyanophénylalanyl)-1 méthyl-4 pipéridine* (II: $NR_2R_3$ = méthyl-4 pipéridino)

Préparé selon le mode opératoire décrit dans l'exemple 4, à partir de la méthyl-4 [Nα-(terbutyloxycarbonyl) p-cyanophénylalanyl]-1 pipéridine (exemple 3).

Cristaux blancs, rendement: 97%, F = 210°C (isopropanol), chlorhydrate.

Exemple 6

*[Nα-(Nα-bétanaphtylsulfonyl-(R)-phénylalanyl) p-cyanophénylalanyl]-1 pipéridine* (V: Ar = β-naphtyle; $R_1$=$CH_2$—$C_6H_5$; $NR_2R_3$=pipéridino)

A une suspension de 12,5 g (0,0425 mole) de chlorhydrate de (p-cyanophénylalanyl)-1 pipéridine dans 200 ml de dichlorométhane, on ajoute successivement, 15,1 g (0,0425 mole) de Nα-(bétanaphtylsulfonyl) (R)-phénylalanine, 4,3 g (0,0425 mole) de triéthylamine, 6,5 g (0,0425 mole) d'hydroxy-1 benzotriazole (HOBT). On refroidit le milieu réactionnel entre 0°C et 5°C et ajoute, goutte à goutte, 8,8 g (0,0425 mole) de

N,N-dicyclohexylcarbodiimide (DCC) dissoute dans 50 ml de dichlorométhane. On abandonne le milieu réactionnel, sous bonne agitation, à température ambiante, pendant 17 h. On filtre le précipité de dicyclohexylurée et le filtrat organique est lavé avec une solution aqueuse saturée de bicarbonate de sodium. La phase organique, séchée sur sulfate de sodium anhydre est évaporée à sec. L'évaporation du solvant laisse un résidu qui est trituré avec de l'acétate d'éthyle. Les cristaux blancs sont filtrés et lavés avec l'éther diisopropylique.

Cristaux blancs, rendement: 64%, F=173°C (acétate d'éthyle).

*Les exemples 7 à 12* utilisent le même mode opératoire que celui décrit dans l'exemple 6. Ils conduisent aux nitriles de formule (V) et résultent du couplage des synthons de formule générale (II) avec les acides de formule générale (III), préalablement activés par transformation de la fonction acide en fonction ester activé (IV) utilisant le réactif de couplage non racémisant DCC/HOBT. Ils sont regroupés dans le tableau ci-après.

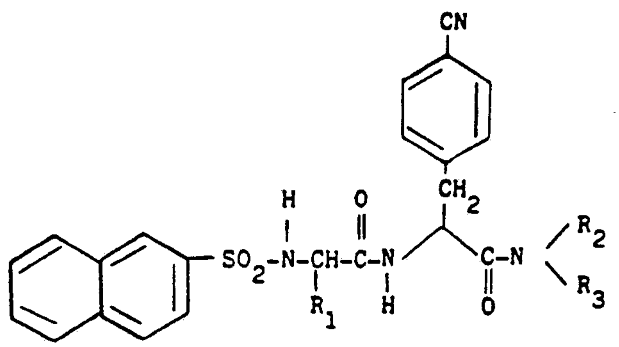

(V : Ar = β-naphtyle)

| Exemple | $R_1$ (configuration de l'aminoacide) | $NR_2R_3$ | Rendement | F°C |
|---|---|---|---|---|
| 7 | $CH_3$ (R) | 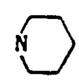 | 51 % | 160° |
| 8 | $C_6H_5$ (R) | 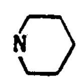 | 38 % | 194° |
| 9 | $CH_2OH$ (S) | 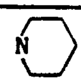 | 75 % | 97° |
| 10 | $CH_3$ (R) | 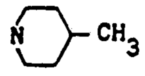 | 73 % | 160° |
| 11 | $CH_3$ (S) | 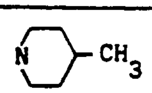 | 72 % | 150° |
| 12 | $CH(CH_3)OH$ (S) | 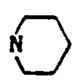 | 78 % | 106° |

Exemple 13

*[Nα-(Nα-bétanaphtylsulfonyl-(S)-alanyl) p-cyanophénylalanyl]-1 pipéridine:* (V: Ar=β-naphtyle; $R_1=CH_3$; $NR_2R_3$=pipéridino)

A une solution de 7,5 g (0,027 mole) de Nα-(bétanaphtylsulfonyl)-(S)-alanine (III: $R_1=CH_3$, Ar=β-naphtyl sulfonyle) de configuration S dans 300 ml d'acétonitrile, on ajoute successivement 11,9 g (0,027 mole) d'hexafluorophosphate de benzotriazolyl-1 oxytris (diméthylamino) phosphonium (BOP), 6,9 g (0,027 mole) de (p-cyanophénylalanyl)-1 pipéridine, (II: $NR_2R_3$=pipéridino) 2,75 g (0,027 mole) de triéthylamine. On abandonne le milieu réactionnel, sous atmosphère inerte, sous bonne agitation, à température ambiante, pendant 20 h. On dilue le milieu réactionnel avec de l'acétate d'éthyle et le lave successivement avec une solution aqueuse saturée de chlorure de sodium, une solution d'acide chlorhydrioque 2N, de l'eau, une solution aqueuse saturée de bicarbonate de sodium puis de l'eau. La phase organique est séchée sur sulfate de sodium anhydre et évaporée à sec. Le résidu est purifié par

chromatographie sur colonne de silice (élution : toluène-acétate d'éthyle 1:1). On récupère des cristaux blancs que l'on sèche.
Cristaux blancs, rendement: 45%; F=155°C.

Exemple 14

*[Nα-(Nα-bétanaphtylsulfonyl-(RS)-phénylglycyl) p-cyanophénylalanyl]-1 pipéridine:* (V: Ar=β-naphtyle; $R_1$=$C_6H_5$; $NR_2R_3$=pipéridino)

On porte au reflux, pendant une heure, sous atmosphère inerte, 3.4 g (0,01 mole) de Nα-bétanaphtylsulfonylphénylglycine (III: $R_1$=$C_6H_5$; Ar=β-naphtyle) racémique (RS) dans 30 ml de chlorure de thionyle. On évapore à ces le milieu réactionnel et dissout le résidu huileux dans 50 ml de dichlorométhane. La solution de chlorure d'acide (IV: $R_1$=$C_6H_5$. Ar=β-naphtyle; R=Cl) dans le dichlorométane est ajoutée goutte à goutte, sous atmosphère inerte, à une solution de 1,3 g (0,005 mole) de (p-cyanophénylalanyl)-1 pipéridine (II: $NR_2R_3$=pipéridino) et de 0,6 g (0,005 mole) de triéthylamine dans 20 ml de dichlorométhane, qui a été préalablement refroidie entre 0° et +5°C. On abandonne le milieu réactionnel à température ambiante pendant 20 h. Les sels insolubles sont filtrés et le filtrat est évaporée à sec. On reprend le résidu par de l'acide chlorhydrique 1N et extrait la phase aqueuse acide obtenue par du dichlorométhane. Les extraits organiques sont séchés sur sulfate de sodium anhydre et évaporés à sec. Le résidu obtenu après évaporation est purifié par chromatographie sur colonne de silice (élution: toluène-acétate d'éthyle 1:1). On obtient des cristaux blancs.
Cristaux blancs, rendement: 80%; F = 190°C

Exemple 15

*[Nα-(Nα-bétanaphtylsulfonyl-(RS)-valyl) p-cyanophénylalanyl]-1 pipéridine* (V: Ar=β-naphtyle; $R_1$=$CH(CH_3)_2$; $NR_1R_2$=pipéridino)

A une suspension de 7 g (0,023 mole) de N-bétanaphtylsulfonylvaline

(III: $R_1$ = $CH(CH_3)_2$; Ar=β-naphtyle) racémique (RS) dans 100 ml de dichlorométhane, maintenue entre 0° et +5°C, on ajoute 2,6 g (0,023 mole) de triéthylamine, puis goutte à goutte 3,4 g (0,025 mole) de chloroformate d'isobutyle et on abandonne une heure à cette température. On ajoute alors 6,2 g (0,024 mole) de (p-cyanophénylalanyl)-1 pipéridine (II: $NR_2R_3$=pipéridino) dissoute dans 50 ml de dichlorométhane et on abandonne à température ambiante le milieu réactionnel pendant 20 h. On évapore à sec, reprend le résidu par de l'eau. La phase aqueuse est extraite par du dichlorométhane. Les extraits organiques sont séchés sur sulfate de sodium anhydre et évaporés à sec. Le résidu huileux est purifié par chromatographie sur colonne de silice (élution: toluène-acétate d'éthyle 1:1).
Cristaux blancs, rendement: 78%; F=178°C

Exemple 16

[Nα-(Nα-bétanaphtylsulfonyl-(R)-phénylglycyl) p-amidinophénylalanyl]-1 pipéridine: (I: Ar=β-naphtyle; $R_1$=$C_6H_5$; $NR_2R_3$=pipéridino) dérivé N°1

*a) Formation de l'imido-ester*

On sature à 0°C, sous atmosphère inerte, 100 ml de méthanol avec du gaz chlorhydrique et ajoute en une seule fois, 8,1 g (0,0139 mole) de [Nα-(Nα-bétanaphtylsulfonyl-R-phénylglycol) p-cyanophénylalanyl]-1 pipéridine (exemple 8) et on abandonne à 0°C pendant 20 h. On évapore à sec, sans chauffer le méthanol et obtient une résine blanche, constituée du chlorhydrate de l'imido-ester de formule générale (VII: Ar=β-naphtyle; $R_1$=$C_6H_5$; $NR_2R_3$=pipéridino), qui est utilisé sans autre purification dans l'étape ultérieure.

b) *Formation de l'amidine*

On sature à des températures comprises entre 0° et +5°C, sous atmosphère inerte, 100 ml de méthanol avec de l'ammoniac gazeux, et ajoute à cette solution méthanolique ammoniacale, la résine blanche, obtenue dans l'exemple 10a, après dissolution dans 20 ml de méthanol. On porte au reflux le mélange réactionnel, sous atmosphère inerte, pendant 3 h. On évapore à sec et reprend le résidu par de l'acide chlorhydrique 1N en excès. La phase aqueuse acide est extraite par le dichlorométhane. La phase organique est séchée sur du sulfate de sodium anhydre et évaporée à sec. On reprend le résidu semi-crisallin, obtenu après évaporation, par de l'eau. Les cristaux insolubles dans l'eau sont récupérés, séchés puis traités par de l'acétate d'éthyle, porte au reflux pendant 10 minutes. Les cristaux insolubles dans l'acétate d'éthyle bouillant sont filtrés et séches.
Cristaux blancs, chlorhydrate hydraté, rendement: 84%; F=188°C

*Les exemples 17 à 23* utilisent les mêmes modes opératoires que ceux décrits dans l'exemple 16. Ils conduisent aux Nα-arylsulfonylaminoacyl p-amidino phénylalaninamides de formule générale (I) et

résultent de la transformation des nitriles de formule générale (V) par l'intermédiaire des imido-esters de formule générale (VI). Ils sont regroupés dans le tableau ci-après.

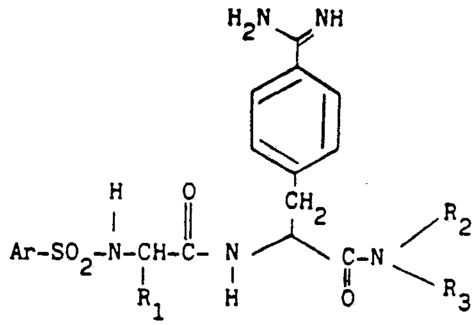

,HCl, $xH_2O$

| exemple | Dérivé | Ar | $R_1$ (configuration de l'aminoacide) | $NR_2R_3$ | x | Rendement | F°C |
|---|---|---|---|---|---|---|---|
| 17 | 2 | 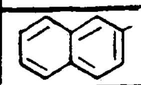 | $CH_2-C_6H_5$(R) | 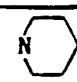 | 1 | 83 % | 178° |
| 18 | 3 | 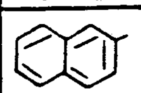 | $CH_3$(R) | 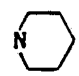 | 1,5 | 53 % | 170° |
| 19 | 4 | 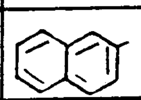 | $CH_3$(S) | 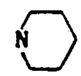 | 1 | 54 % | 176° |
| 20 | 5 | 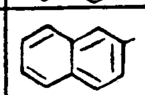 | $CH_3$(R) | 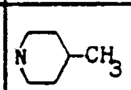 | 2 | 69 % | 177° |
| 21 | 6 | 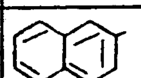 | $CH_3$(S) | 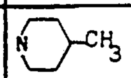 | 2 | 64 % | 176° |
| 22 | 7 | 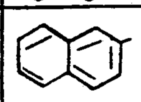 | $CH_2OH$(S) | 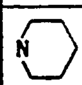 | 1 | 51 % | 162° |
| 23 | 8 | 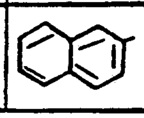 | $CH(CH_3)CH$(S) | 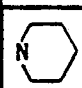 | 0,5 | 68 % | 184 ° |

Les résultats des études toxicologique et pharmacologique qui sont rapportées ci-dessous, ont mis en évidence les intéressantes propriétés des composés de l'invention.

Ces derniers sont doués d'une très bonne activité inhibitrice de la thrombine et possèdent, en outre, de remarquables propriétés antithrombotiques in vivo que ne manifestent pas les composés des formules (A) et (B) et le composé (C).

Comparés à l'héparine, ils ont une durée d'action bien supérieure sans induire d'augmentation du temps de saignement.

L'invention a donc encore pour objet un médicament présentant, en particulier, des propriétés antithrombotiques, caractérisé en ce qu'il contient, à titre de principe actif, un composé de formule (I) ou un sel d'addition avec un acide minéral ou organique thérapeutiquement acceptable.

Etude toxicologique

Les composés de l'invention bénéficient d'une bonne tolérance et d'une faible toxicité.

Les essais effectués sur différentes espèces animales sur les toxicités aigue, subchronique, et chronique, n'ont pas mis en évidence une quelconque réaction locale ou générale, perturbation ou anomalie dans les examens biochimiques, macroscopiques et microscopiques effectués tout au long des essais.

Etude pharmacologique

Dans cette étude, les composés de l'invention ont été comparés à l'héparine et à la [Nα-(Nα-bétanaphtylsulfonylglycyl) p-amidinophénylalanyl]-pipéridine, composé de structure proche décrit comme

un puissant inhibiteur de la thrombine (J. HAUPTMANN et Coll., Thromb., Res., *39*, 771—775, 1983) et qui sera nommé dérivé C.

1°) Temps de thrombine

Le temps de coagulation du plasma citraté en présence de thrombine est mesuré ex vivo chez le rat, selon la technique de BIGGS R. M. (Human blood coagulation, Haemostasis and Thrombosis; Oxford, Blackwell Scientific Publications, 1972).

Les prélèvements sont effectués une heure après administration sous-cutanée du composé à tester, par ponction à l'aorte abdominale. Le sang est recueilli sur citrate de sodium à 3,8% (1 volume pour 9 volumes de sang). Le plasma est obtenu par centrifugation à 2600 g pendant 10 minutes. A 0,2 ml du plasma, on ajoute 0,2 ml d'une solution de thrombine (20 U/ml).

Le temps de coagulation est enregistré.

Les résultats sont rassemblés dans le tableau ci-après et exprimés en secondes.

| Produit | Dose mg/Kg | Voie | Résultat (temps en secondes) | % allongement | p |
|---|---|---|---|---|---|
| témoin | | s.c. | 6 ± 0 | | |
| héparine | 10 | s.c. | 20 ± 4 | 230 | 0,001 |
| témoin | | s.c. | 7 ± 0 | | |
| dérivé C | 10 | s.c. | 9 ± 0 | 29 | 0,001 |
| témoin | | s.c. | 5,9 ± 0 | | |
| dérivé N° 7 | 10 | s.c. | 48,6 ± 10,8 | 782 | 0,001 |
| témoin | | s.c. | 8,2 ± 0,2 | | |
| dérivé N° 4 | 10 | s.c. | 49,9 ± 6,2 | 508 | 0,001 |
| témoin | | s.c. | 7,7 ± 0,1 | | |
| dérivé N° 6 | 10 | s.c. | 12,7 ± 0,3 | 65 | 0,001 |
| témoin | | s.c. | 7 ± 0 | | |
| dérivé N° 8 | 10 | s.c. | 87 ± 11 | 1104 | 0,01 |

2°) Thrombose veineuse à la vrille

Les essais ont été effectués selon une adaptation de la méthode de T. KUMADA et al.; (Thromb. Res., *18*, 189—203, 1980).

Une spirale métallique (bourre-pâte de dentiste recoupée) est insérée dans la veine cave inférieure du rat anesthésié. Une heure auparavant, les animaux ont reçu par la voie sous-cutanée, le composé à tester. Cinq heures après, la spirale est retirée avec le thrombus qu'elle retient, puis séchée par tamponnements

répétés sur papier-filtre et pesée. La spirale est ensuite débarassée du thrombus, séchée et pesée à nouveau. La différence pondérale donne le poids du thrombus.

Les résultats sont rassemblés dans le tableau ci-après:

| Produit | Dose mg/Kg | Poids du thrombus en mg | Variation | p |
|---|---|---|---|---|
| témoin | | 4,47 ± 0,51 | | |
| héparine | 5 | 2,91 ± 0,53 | -35 % | 0,05 |
| héparine | 10 | 1,62 ± 0,34 | -64 % | 0,001 |
| héparine | 20 | 0,26 ± 0,04 | -94 % | 0,001 |
| témoin | | 4,77 ± 0,47 | | |
| dérivé C | 20 | 3,99 ± 0,45 | -16 % | n.s. |
| dérivé C | 50 | 3,63 ± 0,37 | -24 % | n.s. |
| dérivé C | 100 | 3,08 ± 0,28 | -35 % | 0,01 |
| témoin | | 3,81 ± 0,38 | | |
| dérivé N° 4 | 5 | 2,67 ± 0,22 | -30 % | 0,05 |
| dérivé N° 4 | 10 | 2,48 ± 0,35 | -35 % | 0,05 |
| dérivé N° 4 | 20 | 1,92 ± 0,14 | -50 % | 0,001 |
| témoin | | 3,72 ± 0,33 | | |
| dérivé N° 4 | 50 | 2,62 ± 0,26 | -30 % | 0,05 |
| témoin | | 3,72 ± 0,33 | | |
| dérivé N° 7 | 10 | 2,42 ± 0,26 | -35 % | 0,01 |
| témoin | | 4,28 ± 0,53 | | |
| dérivé N° 8 | 10 | 2,07 ± 0,12 | -52 % | 0,01 |

3°) Temps de saignement

Cette étude a été effectuée selon une adaptation de la technique de L. STELLA et al. (Thromb. Res.; 1975, *7*, 709—716).

Après anesthésie du rat au pentobarbital, la queue est sectionnée à 5 mm de l'extrémité et le sang de la blessure soigneusement tamponné toutes les 15 secondes, à l'aide d'un papier filtre jusqu'à hémostase.

Celle-ci est atteinte lorsqu'aucune tache n'apparaît pendant une minute. Les produits à tester sont administrés par la voie sous-cutanée, une heure avant la section de la queue.

Les résultats sont rassemblés dans le tableau ci-après:

| Produit | Dose mg/Kg | Durée en secondes | Extrêmes | p |
|---|---|---|---|---|
| témoin | | 360 | 330-480 | |
| héparine | 5 | 465 | 330-540 | n.s. |
| héparine | 10 | 3600 | 525-> 3600 | 0,01 |
| héparine | 20 | 3600 | 690-> 3600 | 0,01 |
| témoin | | 540 | 405-675 | |
| dérivé C | 10 | 375 | 360-510 | n.s. |
| dérivé C | 20 | 750 | 420-960 | n.s. |
| dérivé C | 50 | 600 | 435-615 | n.s. |
| témoin | | 405 | 390-510 | |
| dérivé N° 4 | 10 | 440 | 400-520 | n.s. |
| dérivé N° 4 | 20 | 450 | 420-530 | n.s. |
| dérivé N° 4 | 50 | 480 | 420-3500 | n.s. |
| témoin | | 540 | 400-680 | |
| dérivé N° 6 | 10 | 380 | 340-480 | n.s. |
| dérivé N° 6 | 20 | 550 | 420-700 | n.s. |
| dérivé N° 6 | 50 | 600 | 430-605 | n.s. |
| témoin | | 525 | 450-610 | |
| dérivé N° 7 | 10 | 605 | 530-760 | n.s. |
| dérivé N° 7 | 20 | 585 | 495-860 | n.s. |
| dérivé N° 7 | 50 | 540 | 460-650 | n.s. |

Temps de saignement: cette étude a nettement montré le risque hémorragique induit par l'héparine qui allonge considérablement le temps de saignement. Etant sans action sur le temps de saignement, les dérivés de l'invention possèdent une marge de sécurité très supérieure à celle de l'héparine.

Le médicament de l'invention peut être présenté pour l'administration orale sous forme de comprimés, comprimés dragéifiés, capsules, gouttes, sirop ou granulés.

Il peut aussi être présenté pour l'administration rectale sous forme de suppositoires et pour l'administration parentérale sous forme de soluté injectable.

Chaque dose unitaire contient avantageusement de 0,005 g à 0,500 g de principe actif en fonction de l'âge du malade et de la gravité de l'affection traitée.

On donnera ci-après, à titre d'exemples non limitatifs, quelques formulations pharmaceutiques du médicament de l'invention.

| | |
|---|---|
| 1) comprimés<br>dérivé N° 4<br>excipient | 0,050 g<br>lactose, amidon de maïs, talc, silice colloïdale, stéarate de magnésium. |
| 2) comprimés dragéifiés<br>dérivé N° 6<br>excipient | 0,025 g<br>sucre, amidon de maïs, polyvinyle pyrrolidone, acide silicique, stéarate de magnésium, talc, jaune orangé. |
| 3) capsules<br>dérivé N° 3<br>excipient | 0,100 g<br>talc, amidon de blé, stéarate de magnésium. |
| 4) suppositoires<br>dérivé N° 5<br>excipient | 0,050 g<br>glycérides semi-synthétiques. |
| 5) soluté injectable<br>dérivé N° 11<br>excipient | 0,025<br>solvant isotonique q.s.p. 5 ml |

Pour ses propriétés anticoagulante et antithrombotique, dépourvu des effets secondaires dus au risque hemorragique, le médicament de l'invention est utilement administré dans la prévention et le traitement de la maladie thromboembolique.

**Revendications pour les Etats contractants: BE CH DE FR GB IT LI LU NL SE**

1. Composés de formule dans laquelle

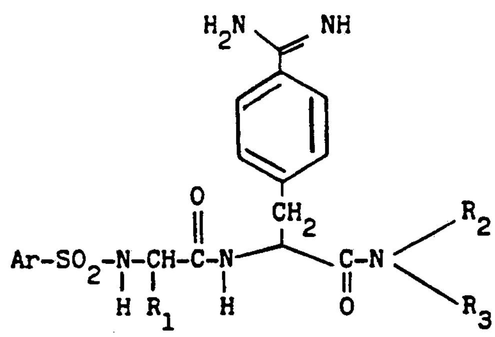

(I)

$R_1$ représente un groupe alcoyle en $C_1$—$C_6$, hydroxyalcoyle en $C_1$—$C_6$, benzyle, un groupe phényle ou un groupe hydroxy-4 phényle;

$R_2$ et $R_3$, identiques ou différents, représentent chacun un radical alcoyle, hydroxyalcoyle, alcényle, ou alcynyle ayant jusqu'à 6 atomes de carbone, ou

forment ensemble avec l'azote auquel ils sont attachés, un hétérocycle saturé choisi parmi morpholino, thiomorpholino, pyrrolidino non substitué ou substitué par un groupe alcoxy (en $C_1$—$C_6$) carbonyle ou carboxyle, pipérazino, (alcoyle en $C_1$—$C_6$)-4 pipérazino, (hydroxyalcoyle en $C_1$—$C_6$)-4 pipérazino, ou pipéridino non substitué ou substitué par un groupe alcoyle en $C_1$—$C_6$, benzyle, hydroxy, hydroxyalcoyle en $C_1$—$C_6$ amino, aminoalcoyle en $C_1$—$C_6$ alcoxy en $C_1$—$C_6$ carbonyle ou carboxyle;

Ar représente un groupe phényle, alpha-naphtyle ou béta-naphtyle ou bien un groupe hétéroaryle choisi parmi pyridyle, quinoléinyle, isoquinoléinyle, et leurs sels d'addition avec les acides minéraux ou organiques pharmaceutiquement acceptables ainsi que les isomères ou leurs mélanges.

2. Composés selon la revendication 1, de formule (I) dans laquelle $R_1$ représente un radical alcoyle ou hydroxyalcoyle.

3. Composés selon la revendication 1 ou 2, de formule (I) dans laquelle le groupe

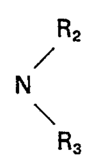

représente un radical pipéridino ou méthyl-4 pipéridino.

4. Composés selon la revendication 1, 2 ou 3, de formule (I) dans laquelle Ar représente un radical naphtyle.

5. [Nα-(Nα-bétanaphtylsulfonyl-(S)-alanyl) p-amidinophénylalanyl]-1 pipéridine et ses sels pharmaceutiquement acceptables.

6. [Nα-(α-bétanaphtylsulfonyl-(S)-alanyl) p-amidinophénylalanyl]-1 méthyl-4 pipéridine et ses sels pharmaceutiquement acceptables.

7. [Nα-(Nα-bétanaphtylsulfonyl-(S)-séryl) p-amidinophénylalanyl]-1 pipéridine et ses sels pharmaceutiquement acceptables.

8. [Nα-(Nα-bétanaphtylsulfonyl-(S)-threonyl) p-amidinophénylalanyl]-1 pipéridine et ses sels pharmaceutiquement acceptables.

9. Procédé de préparation des composés selon les revendications 1 à 8 caractérisé en ce que l'on fait réagir sur la cyano-4 phénylalaninamido de formule (II)

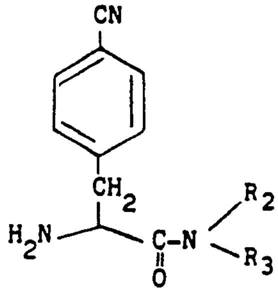

(II)

dans laquelle $R_2$ et $R_3$ ont les mêmes significations que dans la formule (I), un acide de formule

Ar—$SO_2$—NH—CH—COOH
|
$R_1$ (III)

sous sa forme activée (III)

Ar—SO—NH—CH—CO—R
|
$R_1$ (IV)

dans laquelle Ar et $R_1$ ont les mêmes significations que dans la formule (I) et R représente un bon groupement nucléofuge, tel que alcoxycarbonyloxy ou hétéroaryle pour obtenir le composé de formule (V):

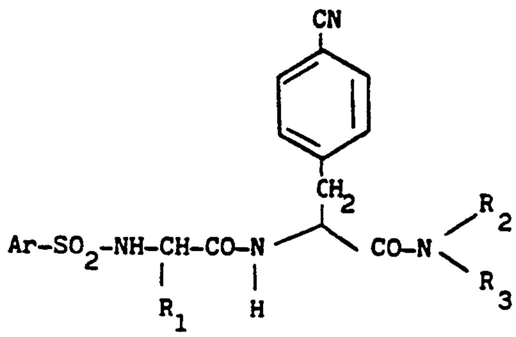

(V)

dans laquelle Ar, $R_1$, $R_2$ et $R_3$ ont les mêmes significations que dans la formule (I), qu'on traite avec un excès d'une solution saturée de gaz chlorhydrique dans un alcool de formule X—OH dans laquelle X représente un radical alcoyle inférieur pour obtenir l'imido-ester de formule (VI) sous forme de chlorhydrate:

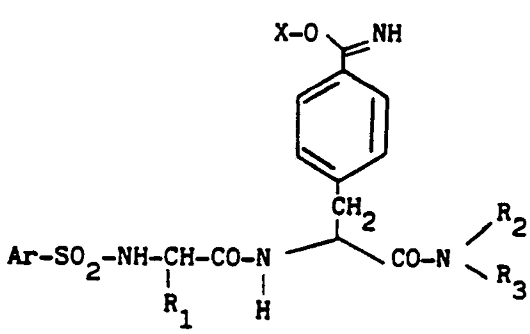

(VI)

dans laquelle Ar, $R_1$, $R_2$, $R_3$ et X ont les mêmes significations précitées, qui est alors traité par un excès d'une solution de gaz ammoniac dans un alcool inférieur à la température d'ébullition du mélange réactionnel pour obtenir le composé de formule (I) recherché.

10. Procédé selon la revendication 9 caractérisé en ce que l'activation de l'acide de formule (III) est effectuée par action d'un chloroformate d'alcoyle.

11. Procédé selon la revendication 9 caractérisé en ce que l'activation de l'acide de formule (III) est effectuée par action de réactifs de couplage n'induisant pas la racémisation, tels que l'hydroxy-1 benzotriazole en présence de N,N-dicyclohexylcarbodiimide, l'hexafluorophosphate de benzotriazolyl-1 oxytris (diméthylamino) phosphonium ou le chlorure de N,N-bis (oxo-2 oxazolidinyl-3) phosphorodiamidique.

12. Procédé selon l'une des revendications 9 à 11 caractérisé en ce que la préparation de l'imido-ester de formule (VI) s'effectue en milieu alcoolique, en présence d'un acide fort.

13. Procédé selon l'une des revendications 9 à 12 caractérisé en ce que la formation de l'amidine de formule (I) s'effectue en milieu alcoolique par action du gaz ammoniac.

14. Médicament caractérisé en ce qu'il contient, à titre de principe actif, un dérivé de formule (I) suivant l'une des revendications 1 à 8 ou l'un de ses sels pharmaceutiquement acceptable.

15. Médicament selon la revendication 14, caractérisé en ce que chaque dose unitaire contient de 0,005 g à 0,500 g de principe actif.

**Revendications pour les Etats contractants: AT GR ES**

1. Procédé de préparation de composés de formule:

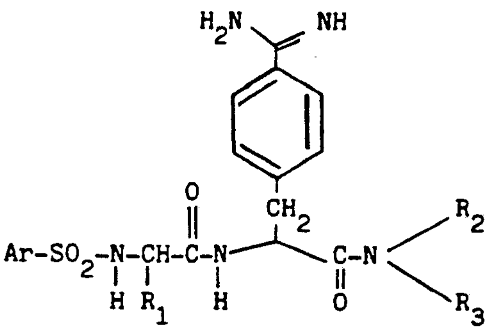

(I)

dans laquelle:

$R_1$ représente un groupe alcoyle en $C_1$—$C_6$, hydroxyalcoyle en $C_1$—$C_6$ benzyle, un groupe phényle ou un groupe hydroxy-4 phényle;

$R_2$ et $R_3$, identiques ou différents, représentent chacun un radical alcoyle, hydroxyalcoyle, alcényle, ou alcynyle ayant jusqu'à 6 atomes de carbone, ou

forment ensemble avec l'azote auquel ils sont attachés, un hétérocycle saturé choisi parmi morpholino, thiomorpholino, pyrrolidino non substitué ou substitué par un groupe alcoxy (en $C_1$—$C_6$) carbonyle ou carboxyle, pipérazino, (alcoyle en $C_1$—$C_6$)-4 pipérazino, (hydroxyalcoyle en $C_1$—$C_6$)-4 pipérazino, ou pipéridino non substitué ou substitué par un groupe alcoyle en $C_1$—$C_6$, benzyle, hydroxy, hydroxyalcoyle en $C_1$—$C_6$ amino, aminoalcoyle en $C_1$—$C_6$,alcoxy en $C_1$—$C_6$ carbonyle ou carboxyle;

Ar représente un groupe phényle, alpha-naphtyle ou béta-naphtyle ou bien un groupe hétéroaryle choisi parmi pyridyle, quinoléinyle, isoquinoléinyle, et leurs sels d'addition avec les acides minéraux ou organiques pharmaceutiquement acceptables ainsi que les isomères ou leurs mélanges, caractérisé en ce que l'on fait réagir sur la cyano-4 phénylalaninamido de formule:

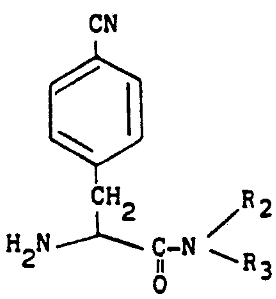

(II)

dans laquelle $R_2$ et $R_3$ ont les mêmes significations que dans la formule (I), un acide de formule:

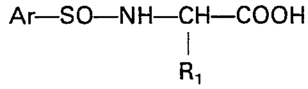

(III)

sous sa forme activée (III)

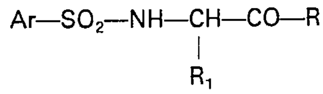

(IV)

dans laquelle Ar et $R_1$ ont les mêmes significations que dans la formule (I) et R représente un bon groupement nucléofuge, tel que alcoxycarbonyloxy ou hétéroaryle pour obtenir le composé de formule:

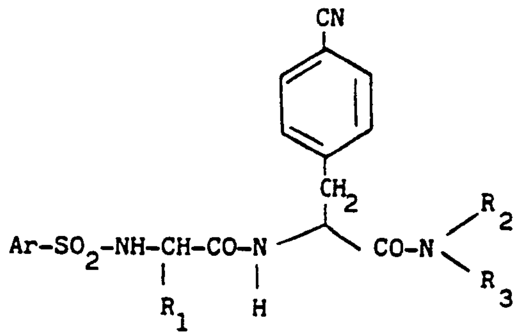

(V)

dans laquelle Ar, $R_1$, $R_2$ et $R_3$ ont les mêmes significations que dans la formule (I), qu'on traite avec un excès d'une solution saturée de gaz chlorhydrique dans un alcool de formule X—OH dans laquelle X représente un radical alcoyle inférieur pour obtenir l'imido-ester de formule (VI) sous forme de chlorhydrate:

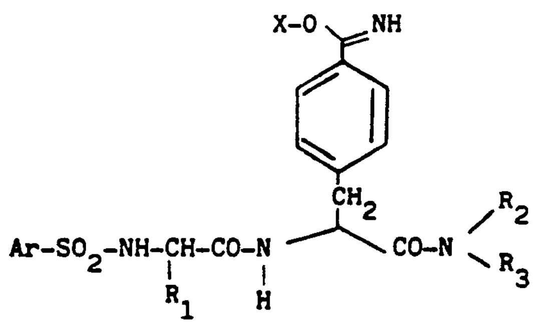

(VI)

dans laquelle Ar, $R_1$, $R_2$, $R_3$ et X ont les mêmes significations précitées, qui est alors traité par un excès d'une solution de gaz ammoniac dans un alcool inférieur à la température d'ébullition du mélange réactionnel pour obtenir le composé de formule (I) recherché.

2. Procédé selon la revendication 1, caractérisé en ce que l'activation de l'acide de formule (III) est effectuée par action d'un chloroformate d'alcoyle.

3. Procédé selon la revendication 1, caractérisé en ce que l'activation de l'acide de formule (III) est effectuée par action de réactifs de couplage n'induisant pas la racémisation, tels que l'hydroxy-1 benzotriazole en présence de N,N-dicyclohexylcarbodiimide, l'hexafluorophosphate de benzotriazolyl-1 oxytris (diméthylamino) phosphonium ou le chlorure de N,N-bis (oxo-2 oxazolidinyl-3) phosphorodiamidique.

4. Procédé selon l'une des revendications 1 à 3, caractérisé en ce que la préparation de l'imido-ester de formule (VI) s'effectue en milieu alcoolique, en présence d'un acide fort.

5. Procédé selon l'une des revendications 1 à 4, caractérisé en ce que la formation de l'amidine de formule (I) s'effectue en milieu alcoolique par action du gaz ammoniac.

6. Procédé de préparation d'un médicament caractérisé en ce que l'on utilise, à titre de principe actif, un dérivé de formule (I) obtenu suivant l'une des revendications 1 à 5 ou l'un de ses sels pharmaceutiquement acceptables.

**Patentansprüche für die Vertragsstaaten: BE CH DE FR GB IT LI LU NL SE**

1. Verbindungen der Formel

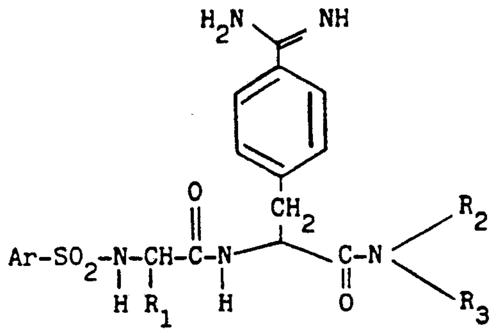

(I)

in der bedeuten:

$R_1$ $C_1$—$C_6$-Alkyl, $C_1$—$C_6$-Hydroxyalkyl, Benzyl, Phenyl oder 4-Hydroxyphenyl;

$R_2$ und $R_3$ zugleich oder unabhängig Alkyl, Hydroxyalkyl, Alkenyl oder Alkinyl mit jeweils bis zu 6 Kohlenstoffatomen

oder

zusammen mit dem Stickstoffatom, an dem sie gebunden sind, einen gesättigten Heterocyclus, der ausgewählt ist unter Morpholino, Thiomorpholino, Pyrrolidino, das ggfs. mit einer Alkoxy-($C_1$—$C_6$)-carbonylgruppe oder einer Carboxylgruppe substituiert ist, Piperazino, 4-($C_1$—$C_6$-Alkyl)-substituiertes Piperazino, 4-($C_1$—$C_6$-Hydroxyalkyl)-substituiertes Piperazino oder Piperidino, das ggfs. mit $C_1$—$C_6$-Alkyl, Benzyl, Hydroxy, $C_1$—$C_6$-Hydroxyalkyl, Amino, $C_1$—$C_6$-Aminoalkyl, Alkoxy-($C_1$—$C_6$)-carbonyl oder Carboxyl substituiert ist,

und

Ar Phenyl, α-Naphthyl oder β-Naphthyl oder eine Heteroarylgruppe, die ausgewählt ist unter Pyridyl, Chinolinyl und Isochinolinyl, sowie ihre Additionssalze mit pharmazeutisch geeigneten anorganischen oder organischen Säuren sowie die Isomeren oder ihre Gemische.

2. Verbindungen der Formel I nach Anspruch 1, in der $R_1$ Alkyl oder Hydroxyalkyl bedeutet.

3. Verbindungen der Formel I nach Anspruch 1 oder 2, in der die Gruppe

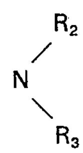

Piperidino oder 4-Methylpiperidino bedeutet.

4. Verbindungen der Formel I nach Anspruch 1, 2 oder 3, in der Ar Naphthyl bedeutet.

5. 1-[Nα-(Nα-β-Naphthylsulfonyl-(S)-alanyl)-p-amidinophenylalanyl]-piperidin sowie seine pharmazeutisch akzeptablen Salze.

6. 1-[Nα-(Nα-β-Naphthylsulfonyl-(S)-alanyl)-p-amidinophenylalanyl]-4-methylpiperidin und seine pharmazeutisch akzeptablen Salze.

7. 1-[Nα-(Nα-β-Naphthylsulfonyl-(S)-seryl)-p-amidinophenylalanyl]-piperidin und seine pharmazeutisch akzeptablen Salze.

8. 1-[Nα-(Nα-β-Naphthylsulfonyl-(S)-threonyl)-p-amidinophenylalanyl]-piperidin und seine pharmazeutisch akzeptablen Salze.

9. Verfahren zur Herstellung der Verbindungen nach den Ansprüchen 1 bis 8, gekennzeichnet durch Umsetzung des 4-Cyanophenylalaninamids der Formel II

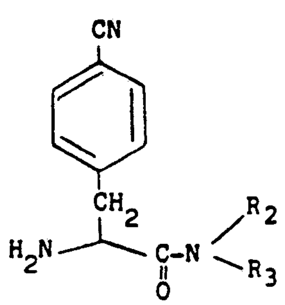

(II)

mit $R_2$ und $R_3$ wie oben in Formel I
mit einer Säure der Formel

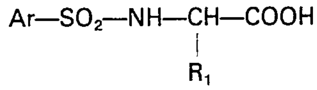

(III)

in ihrer aktivierten Form IV

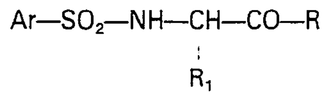

(IV),

in der Ar und $R_1$ dasselbe wie oben in Formel I und
R eine geeignete nucleofolge Gruppe wie Alkoxycarbonyloxy oder Heteroaryl bedeuten,
zur Verbindung der Formel V

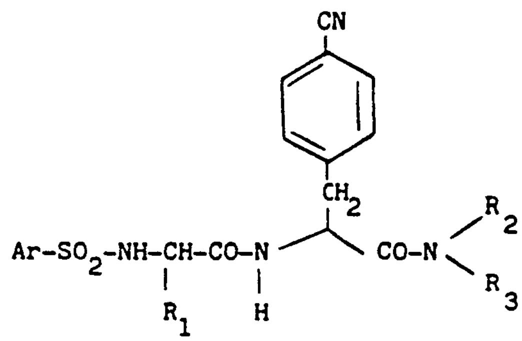

(V)

mit Ar, $R_1$, $R_2$ und $R_3$ wie oben in Formel I, Behandlung der erhaltenen Verbindung mit einem Überschuß einer gesättigten Lösung von gasförmigem Chlorwasserstoff in einem Alkohol der Formel X—OH, in der X eine niedere Alkylgruppe darstellt, unter Erhalt des Imidoesters der Formel VI in Form des Hydrochlorids

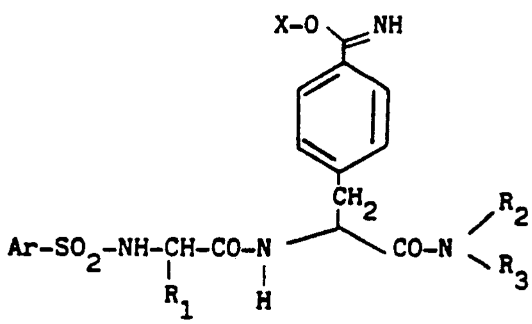

(VI)

mit Ar, $R_1$, $R_2$, $R_3$ und X wie oben und
Behandlung der erhaltenen Verbindung mit einem Überschuß einer Lösung von gasförmigem Ammoniak in einem niederen Alkohol bei der Siedetemperatur des Reaktionsgemischs unter Erhalt der angestrebten Verbindung der Formel I.

10. Verfahren nach Anspruch 9, dadurch gekennzeichnet, daß die Aktivierung der Säure der Formel III durch Einwirkung eines Alkylchlorformiats vorgenommen wird.

11. Verfahren nach Anspruch 9, dadurch gekennzeichnet, daß die Aktivierung der Säure der Formel III durch Einwirkung von Kopplungsmitteln, die nicht zu einer Racemisierung führfen, wie 1-Hydroxybenzotriazol in Gegenwart von N,N-Dicyclohexylcarbodiimid, 1-Benzotriazolyl-oxy-tris(dimethylamino)-phosphoniumhexafluorphosphat oder N,N-Bis(2-oxo-3-oxazolidinyl)-phosphordiamid-chlorid, vorgenommen wird.

12. Verfahren nach einem der Ansprüche 9 bis 11, dadurch gekennzeichnet, daß die Herstellung des Imidoesters der Formel VI in einem alkoholischen Medium in Gegenwart einer starken Säure durchgeführt wird.

13. Verfahren nach einem der Ansprüche 9 bis 12, dadurch gekennzeichnet, daß die Herstellung des Amidins der Formel I in einem alkoholischen Medium durch Einwirkung von Ammoniakgas vorgenommen wird.

14. Pharmazeutische Mittel, dadurch gekennzeichnet, daß sie als Wirkstoff ein Derivat der Formel I nach einem der Ansprüche 1 bis 8 oder eines seiner pharmazeutisch akzeptablen Salze enthalten.

15. Pharmazeutische Mittel nach Anspruch 14, dadurch gekennzeichnet, daß jede Einheitsdosis 0,005 bis 0,500 g Wirkstoff enthält.

**Patentansprüche für die Vertragsstaaten: AT ES GR**

1. Verfahren zur Herstellung von Verbindungen der Formel

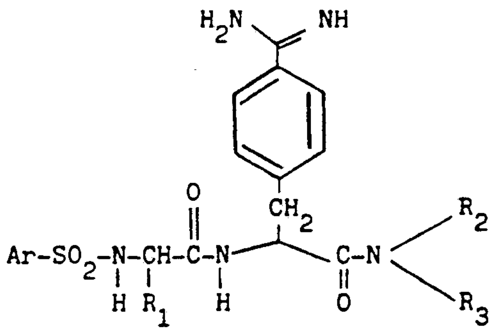

(I)

in der bedeuten:

$R_1$ $C_1$—$C_6$-Alkyl, $C_1$—$C_6$-Hydroxyalkyl, Benzyl, Phenyl oder 4-Hydroxyphenyl;

$R_2$ und $R_3$ zugleich oder unabhängig Alkyl, Hydroxyalkyl, Alkenyl oder Alkinyl mit jeweils bis zu 6 Kohlenstoffatomen

oder

zusammen mit dem Stickstoffatom, an dem sie gebunden sind, einen gesättigten Heterocyclus, der ausgewählt ist unter Morpholino, Thiomorpholino, Pyrrolidino, das ggfs. mit einer Alkoxy-($C_1$—$C_6$)-carbonylgruppe oder einer Carboxylgruppe substituiert ist, Piperazino, 4-($C_1$—$C_6$-Alkyl)-substituiertes Piperazino, 4-($C_1$—$C_6$-Hydroxyalkyl)-substituiertes Piperazino oder Piperidino, das ggfs. mit $C_1$—$C_6$-Alkyl, Benzyl, Hydroxy, $C_1$—$C_6$-Hydroxyalkyl, Amino, $C_1$—$C_6$-Aminoalkyl, Alkoxy-($C_1$—$C_6$)-carbonyl oder Carboxyl substituiert ist,

und

Ar Phenyl, α-Naphthyl oder β-Naphthyl oder eine Heteroarylgruppe, die ausgewählt ist unter Pyridyl, Chinolinyl und Isochinoinyl, sowie ihrer in Additionssalze mit pharmazeutisch akzentablen anorganischen oder organischen Säuren sowie ihrer Isomeren oder ihrer Gemische, gekennzeichnet durch Umsetzung des 4-Cyanophenylalaninamids der Formel II

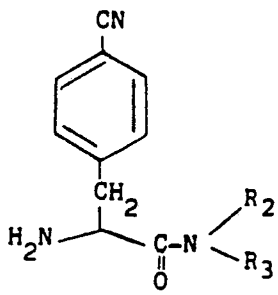

(II)

mit $R_2$ und $R_3$ wie oben in Formel I mit einer Säure der Formel

$$Ar—SO_2—NH—\underset{\displaystyle R_1}{\underset{|}{CH}}—COOH \qquad (III)$$

in ihrer aktivierten Form IV

$$Ar—SO_2—NH—\underset{\displaystyle R_1}{\underset{|}{CH}}—CO—R \qquad (IV),$$

in der Ar und $R_1$ dasselbe wie oben in Formel I und

R eine geeignete nucleotage Gruppe wie Alkoxycarbonyloxy oder Heteroaryl bedeuten, zur Verbindung der Formel V

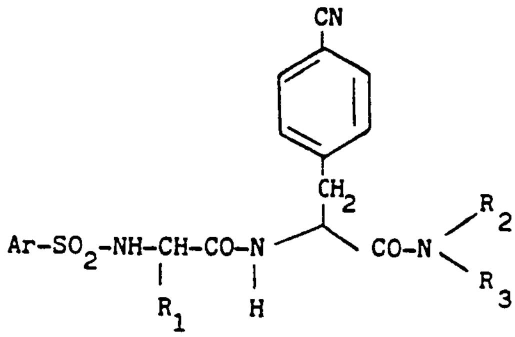

(V)

mit Ar, $R_1$, $R_2$ und $R_3$ wie oben in Formel I, Behandlung der erhaltenen Verbindung mit einem Überschuß einer gesättigten Lösung von gasförmigem Chlorwasserstoff in einem Alkohol der Formel X—OH, in der X eine niedere Alkylgruppe darstellt, unter Erhalt des Imidoesters der Formel VI in Form des Hydrochlorids

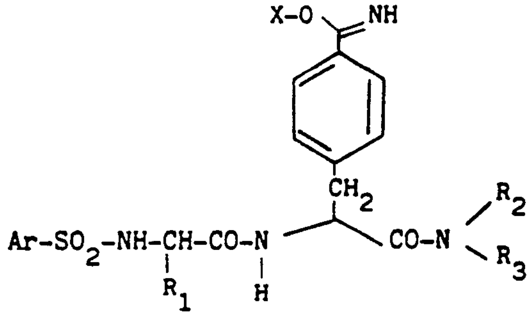

(VI)

mit Ar, $R_1$, $R_2$, $R_3$ und X wie oben und
Behandlung der erhaltenen Verbindung mit einem Überschuß einer Lösung von gasförmigem Ammoniak in einem niederen Alkohol bei der Siedetemperatur des Reaktionsgemischs unter Erhalt der angestrebten Verbindung der Formel I.

2. Verfahren nach Anspruch 1, dadurch gekennzeichnet, daß die Aktivierung der Säure der Formel III durch Einwirkung eines Alkylchlorformiats vorgenommen wird.

3. Verfahren nach Anspruch 1, dadurch gekennzeichnet, daß die Aktivierung der Säure der Formel III durch Einwirkung von Kopplungsmitteln, die nicht zu einer Racemisierung führen, wie 1-Hydroxybenzotriazol in Gegenwart von N,N-Dicyclohexylcarbodiimid, 1-Benzotriazolyl-oxy-tris(dimethylamino)-phosphoniumhexafluorphosphat oder N,N-Bis(2-oxo-3-oxazolidinyl)-phosphordiamid-chlorid, vorgenommen wird.

4. Verfahren nach einem der Ansprüche 1 bis 3, dadurch gekennzeichnet, daß die Herstellung des Imidoesters der Formel VI in einem alkoholischen Medium in Gegenwart einer starken Säure durchgeführt wird.

5. Verfahren nach einem der Ansprüche 1 bis 4, dadurch gekennzeichnet, daß die Herstellung des Amidins der Formel I in einem alkoholischen Medium durch Einwirkung von Ammoniakgas vorgenommen wird.

6. Verfahren zur Herstellung pharmazeutischer Mittel, gekennzeichnet durch Verwendung eines nach einem der Ansprüche 1 bis 5 erhaltenen Derivats der Formel I oder eines seiner pharmazeutisch akzeptablen Salze als Wirkstoff.

**Claims for the Contracting States: BE CH DE FR GB IT LI LU NL SE**

1. Compounds of formula

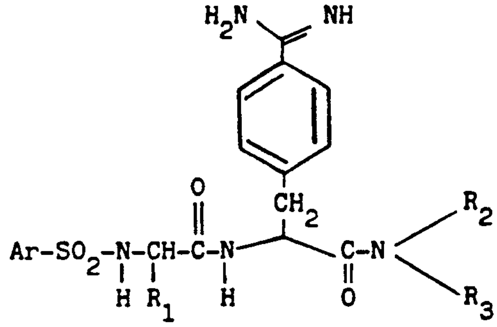

(I)

wherein

$R_1$ represents a $C_{1-6}$ alkyl, $C_{1-6}$ hydroxyalkyl, benzyl, phenyl or 4-hydroxyphenyl group;

$R_2$ and $R_3$, which may be identical or different, each represent an alkyl, hydroxyalkyl, alkenyl or alkynyl group having up to 6 carbon atoms, or form together with the nitrogen to which they are attached a saturated heterocyclic group selected from among thiomorpholino, pyrrolidino, either unsubstituted or substituted by a ($C_{1-6}$ alkoxy)carbonyl or carboxyl group, piperazino, 4-($C_{1-6}$ alkyl)-piperazino, 4-($C_{1-6}$ hydroxyalkyl)-piperazino or piperidino, either unsubstituted or subtituted by a $C_{1-6}$ alkyl, benzyl, hydroxy, $C_{1-6}$ hydroxyalkyl, amino, $C_{1-6}$ aminoalkyl, ($C_{1-6}$ alkoxy)carbonyl or carboxyl group;

Ar represents a phenyl, alpha-naphthyl or beta-naphthyl group or a heteroaryl group selected from among pyridyl, quinolinyl, isoquinolinyl, and the addition salts thereof with pharmaceutically acceptable inorganic or organic acids, as well as the isomers or mixtures thereof.

2. Compounds according to claim 1, of formula (I), wherein $R_1$ represents an alkyl or hydroxyalkyl radical.

3. Compounds according to claim 1 or 2 of formula (I), wherein the group

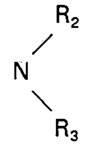

represents a piperidino or 4-methyl-piperidino radical.

4. Compounds according to claim 1, 2 or 3, of formula (I), wherein Ar represents a naphthyl radical.

5. 1-[Nα-(Nα-betanaphthylsulphonyl-(S)-alanyl)-p-amidinophenylalanyl]-piperidine and the pharmaceutically acceptable salts thereof.

6. 1-[Nα-(α-betanaphthylsulphonyl-(S)-alanyl)-p-amidinophenylalanyl]-4-methyl-piperidine and the pharmaceutically acceptable salts thereof.

7. 1-[Nα-(Nα-betanaphthylsulphonyl-(S)-seryl)-p-amidinophenylalanyl]-piperidine and the pharmaceutically acceptable salts thereof.

8. 1-[Nα-(Nα-betanapththylsulphonyl-(S)-threonyl)-p-amidinophenylalanyl]-piperidine and the pharmaceutically acceptable salts thereof.

9. Process for preparing compounds according to claims 1 to 8, characterised in that a 4-cyano-phenylalanine amide of formula (II)

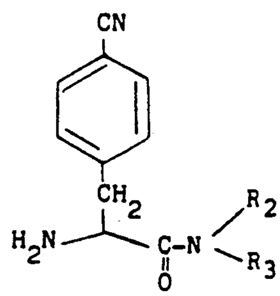

(II)

wherein $R_2$ and $R_3$ have the same meanings as in formula (I), is reacted with an acid of formula

Ar—SO₂—NH—CH—COOH (III)
|
R¹

in its activated form (III)

Ar—SO₂—NH—CH—CO—R (IV)
|
R¹

wherein Ar and $R_1$ have the same meanings as in formula (I) and R represents a suitable nucleofugic group such as alkoxycarbonyloxy or heteroaryl, in order to obtain the compound of formula (V)

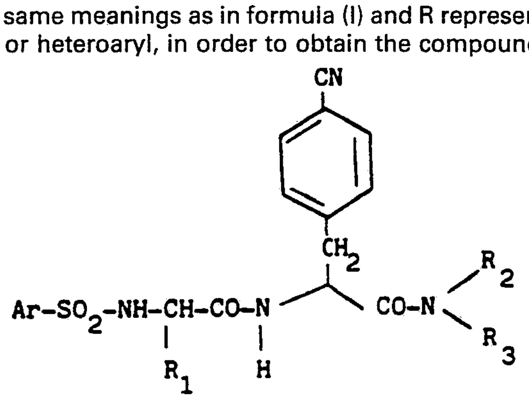

(V)

wherein Ar, $R_1$, $R_2$ and $R_3$ have the same meanings as in formula (I), which is then treated with an excess of a saturated solution of hydrogen chloride gas in an alcohol of formula X—OH wherein X represents a lower alkyl radical, in order to obtain the imido ester of formula (VI) in the form of the hydrochloride:

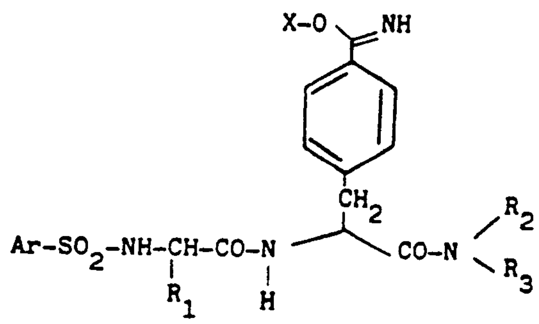

(VI)

wherein Ar, $R_1$, $R_2$, $R_3$ and X have the same meanings as before, which is then treated with an excess of a solution of ammonia gas in a lower alcohol at the boiling temperature of the reaction mixture, in order to obtain the desired compound of formula (I).

10. Process according to claim 9, characterised in that the acid of formula (III) is activated by the action of an alkylchloroformate.

11. Process according to claim 9, characterised in that the acid of formula (III) is activated by the action of coupling reagents which do not cause racemisation, such as 1-hydroxybenzotriazole in the presence of N,N-dicyclohexylcarbodiimide, 1-benzotriazolyloxytris(dimethylamino)-phosphonium hexafluorophosphate or N,N-bis-(2-oxo-3-oxazolidinyl)-phosphorodiamidic chloride.

12. Process according to one of claims 9 to 11, characterised in that the imidoester of formula (VI) is prepared in an alcoholic medium, in the presence of a strong acid.

13. Process according to one of claims 9 to 12, characterised in that the amidine of formula (I) is formed in an alcoholic medium by the action of ammonia gas.

14. Medicament, characterised in that it contains as active principle a derivative of formula (I) acording to one of claims 1 to 8 or one of the pharmaceutically acceptable salts thereof.

15. Medicament according to claim 14, characterised in that each single dose contains from 0.005 g to 0.500 g of active principle.

**Claims for the Contracting States: AT ES GR**

1. Process for preparing compounds of formula

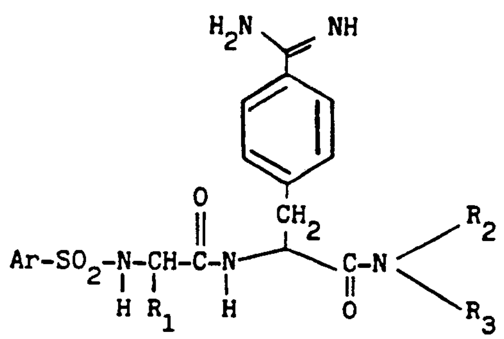

(I)

wherein

$R_1$ represents a $C_{1-6}$ alkyl, $C_{1-6}$ hydroxyalkyl, benzyl, phenyl or 4-hydroxyphenyl group;

$R_2$ and $R_3$, which may be identical or different, each represent an alkyl, hydroxyalkyl, alkenyl or alkynyl group having up to 6 carbon atoms, or form together with the nitrogen to which they are attached a saturated heterocyclic group selected from among thiomorpholino, pyrrolidino, either unsubstituted or substituted by a ($C_{1-6}$ alkoxy)carbonyl or carboxyl group, piperazino, 4-($C_{1-6}$ alkyl)-piperazino, 4-($C_{1-6}$ hydroxyalkyl)-piperazino or piperidino, either unsubstituted or subtituted by a $C_{1-6}$ alkyl, benzyl, hydroxy, $C_{1-6}$ hydroxyalkyl, amino, $C_{1-6}$ aminoalkyl, ($C_{1-6}$ alkoxy)carbonyl or carboxyl group; Ar represents a phenyl, alpha-naphthyl or beta-naphthyl group or a heteroaryl group selected from among pyridyl, quinolinyl, isoquinolinyl, and the addition salts thereof with pharmaceutically acceptable inorganic or

organic acids, as well as the isomers or mixtures thereof, characterised in that a 4-cyano-phenylalanine. amide of formula (II)

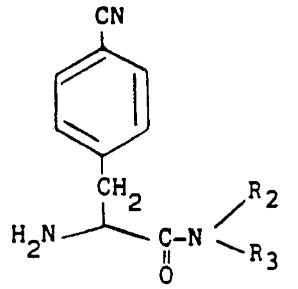

(II)

wherein $R_2$ and $R_3$ have the same meanings as in formula (I), is reacted with an acid of formula

$$Ar—SO_2—NH—CH(R^1)—COOH \qquad (III)$$

in its activated form (III)

$$Ar—SO_2—NH—CH(R^1)—CO—R \qquad (IV)$$

wherein Ar and $R_1$ have the same meanings as in formula (I) and R represents a suitable nucleofugic group such as alkoxycarbonyloxy or heteroaryl, in order to obtain the compound of formula (V)

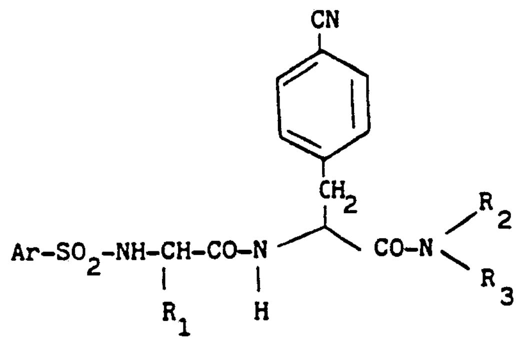

(V)

wherein Ar, $R_1$, $R_2$ and $R_3$ have the same meanings as in formula (I), which is then treated with an excess of a saturated solution of hydrogen chloride gas in an alcohol of formula X—OH wherein X represents a lower alkyl radical, in order to obtain the imido ester of formula (VI) in the form of the hydrochloride:

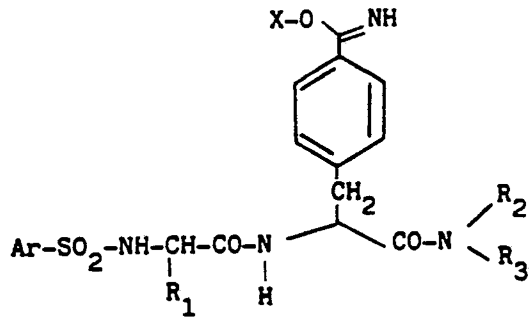

(VI)

wherein Ar, $R_1$, $R_2$, $R_3$ and X have the same meanings as before, which is then treated with an excess of a solution of ammonia gas in a lower alcohol at the boiling temperature of the reaction mixture, in order to obtain the desired compound of formula (I).

2. Process according to claim 1, characterised in that the acid of formula (III) is activated by the action of an alkylchloroformate.

3. Process according to claim 1, characterised in that the acid of formula (III) is activated by the action of coupling reagents which do not cause racemisation, such as 1-hydroxybenzotriazole in the presence of N,N-dicyclohexylcarbodiimide, 1-benzotriazolyloxytris(dimethylamino)-phosphonium hexafluorophosphate or N,N-bis-(2-oxo-3-oxazolidinyl)-phosphorodiamidic chloride.

4. Process according to one of claims 1 to 3, characterised in that the imidoester of formula (VI) is prepared in an alcoholic medium, in the presence of a strong acid.

5. Process according to one of claims 1 to 4, characterised in that the amidine of formula (I) is formed in an alcoholic medium by the action of ammonia gas.

6. Process for preparing a medicament characterised in that a derivative of formula (I) obtained according to one of claims 1 to 5 or a pharmaceutically acceptable salt thereof is used as the active principle.